# EUROPEAN PATENT SPECIFICATION

(11) **EP 2 022 451 B1**
(45) Date of publication and mention of the grant of the patent: **03.11.2010**
(21) Application number: 08161638.5
(22) Date of filing: 01.08.2008
(51) Int. Cl.: A61F 5/44

(54) **Drainable ostomy pouch having flexible stiffening strips with plural grooves**
Entwässerbarer Ostomiebeutel mit flexiblen Versteifungsstreifen mit mehreren Rillen
Poche de stomie vidable disposant de bandes de renforcement avec plusieurs rainures

(30) Priority: 09.08.2007 US 954979 P
(43) Date of publication of application: 11.02.2009
(73) Proprietor: HOLLISTER INCORPORATED, Libertyville, Illinois 60048 (US)
(72) Inventor: Winther, Knud, 3070 Snekkersten (DK)
(74) Representative: Aamand, Jesper L.

(56) References cited:
- EP-A- 1 378 218
- WO-A-03/086250

## Description

The present invention relates a drainable ostomy pouch comprising
- a first, proximal, and a second, distal, side wall of flexible sheet material sealed to each other for defining a cavity therebetween for receiving human stomal discharge through an aperture in said proximal side wall, and a drainage portion extending downwardly in a longitudinal direction thereof and ending in a drainage opening extending in a transverse direction with respect to said longitudinal direction,
- a first, proximal, and a second, distal, elongate, flexible stiffening strip each made of a stiff plastics material and each having a width, a length substantially greater than said width, a first major surface, and a second major surface parallel with and opposed to said first major surface, said stiffening strips extending in said transverse direction in the vicinity of said drainage opening,
- said first major surface of each of said stiffening strips being attached to the outer surface of said first and second side walls, respectively, of said drainage portion such that said first major surfaces of said stiffening strips face each other.

In connection with the use of such ostomy pouches, several considerations are of great importance to the quality of life of the persons utilizing the pouches. The pouches should be easy to drain without risking soiling of clothes or the surroundings, they should be easy to close securely after being drained and they should be amenable to being cleaned after drainage and before closing again such that the risk of leakages and unpleasant odours is substantially reduced.

Many different solutions concerning the closing, cleaning and drainage operations have been proposed and implemented. These solutions all have serious disadvantages regarding security of the closure, requirements to dexterity often not at hand for elderly persons, difficult cleaning procedures, and so on.

UK patent application No. GB 2 000 683 discloses a drainable ostomy pouch of the type in reference having a bar of relatively rigid polymeric material bonded to the outer surface of the distal side wall at the bottom of the drainage portion adjacent and along a drainage aperture extending transversely to the longitudinal direction of the drainage portion. This bar is utilized to facilitate folding of the drainage portion in said longitudinal direction until a transversely extending strip of Velcro attached to the proximal side wall can be engaged by a corresponding strip of Velcro located on a transversely extending flap attached to said distal wall.

The drainage aperture and the area surrounding it are difficult to clean because of the thin proximal wall film not being easy to locate and separate from the bar for cleaning and removing any elongate material between said film and the bar. Furthermore, when folding the drainage portion upwards the end of the proximal wall film may be displaced upwards and wrinkled thereby increasing the risk of a deficient closure with leakage occurring along said wrinkles.

International patent application WO 03/86250 discloses a drainable ostomy pouch of the type in reference having two flexible stiffening strips arranged adjacent to and along a drainage opening at the bottom of a discharge portion of the pouch. As it is difficult to cause the pouch film walls at the discharge opening to separate for cleaning purposes after the contents of the pouch have been discharge because of the tendency of the films to cling to one another because of the remnant moisture on the inner surfaces of the walls, several means are disclosed to facilitate the bending of the stiffening strips away from one another when finger pressure is applied to the ends of the strips. On of such means is that the stiffening strips are curved in the longitudinal direction thereof such that the surface of each strip attached to the corresponding side wall is concave in the longitudinal direction of the strip.

It was thought that as the inherent tendency of the stiffening strips to flex away from one another under the pressure applied longitudinally to the ends of the strips is built into the strips, then the strips would separate under such pressure and open the drainage opening against the clinging force of the film walls.

However, this has turned out to be the case in only very few of the instances where such separation is attempted, the strips flex in the same direction under the pressure of the fingers such that the drainage opening is not opened. This presumably is due to that the adherence of the film walls to one another is stronger than the tendency of the strips to flex away from each other and the strips therefore are held together until they flex in the same direction.

UK patent GB 2 388 322 B discloses a drainable pouch of the type in reference where each of the two elongate strips is provided with a fold or crease extending transversely to the longitudinal direction of the strips. The purpose of the fold or crease is that each strip should fold about its respective crease when the strips are compressed in the longitudinal direction so as to pull the film walls apart.

However, it has also in this case turned out that only in a very few of the instances where such separation is attempted, the strips flex in the same direction when compressed in the longitudinal direction such that the drainage opening is not opened. This presumably is also due to the adherence of the film walls to one another is stronger than the tendency of the strips to flex away from each other by folding around their respective creases and the strips therefore are held together until they flex in the same direction.

The object of the present invention is to provide a drainable ostomy pouch where the opening of the drainage aperture for cleaning of the aperture and the region surrounding it is rendered easier to carry out and thereby provides a more secure closure of the pouch after being emptied.

According to the invention, this object is achieved by said first major surface of each of said stiffening strips being provided with at least two spaced grooves extending across said width of said stiffening strips.

Experiments have shown that by these means the percentage of cases where the strips flex away from each other when finger pressure is applied to the ends of the strips is higher than with the prior art strips.

The percentage of cases where the strips flex away from each other increases when the number of grooves is increased. Whenl five spaced grooves are provided on the strips the percentage of cases is so high that satisfactory cleaning is achieved in nearly all cases.

The grooves may be score lines, and the score lines may be provided by scoring said first major surface with a sharp, hard tool while said second surface is supported by a resilient surface.

The grooves may be pressure lines, and the pressure lines may be provided by pressing a hard tool with a sharp edge against said first major surface while said second surface is supported by a resilient surface.

In a currently preferred embodiment of a pouch according to the invention, each of said stiffening strips is arcuate in said transverse direction such that said first major surface is concave in said transverse direction.

In the following, the invention will be explained more in detail in connection with a currently preferred embodiment thereof shown, solely by way of example, in the accompanying drawings, where
Figs. 1 and 2 are schematic plan views of the proximal and distal sides, respectively, of a currently preferred embodiment of a pouch according to the invention,
Fig. 3 is a schematic enlarged scale cross-sectional view of the elongate drainage portion of the pouch of Figs. 1-2 taken along line A-A in Figs. 1 and 2, and
Fig. 4 is a schematic, enlarged scale cross-sectional view taken along line B-B in Fig. 3, with the thicknesses are greatly exaggerated for the sake of clarity.

The ostomy pouch according to the invention may be part of a one-piece or of a two-piece ostomy appliance.

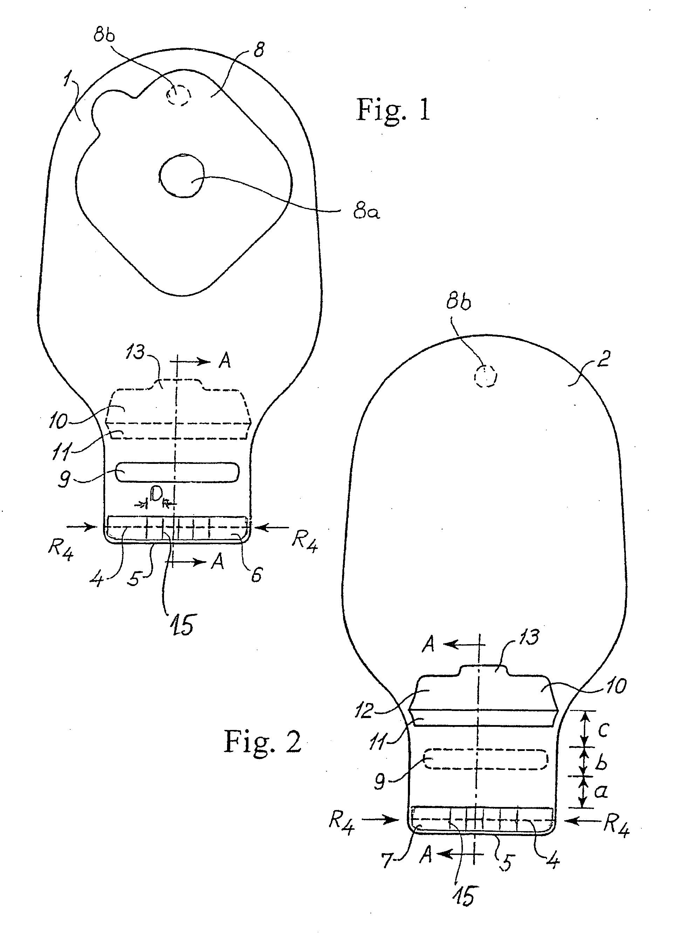

Referring now to Figs 1-3, the currently preferred embodiment of an ostomy pouch according to the invention comprises three sheets or films of a liquid and gas impermeable flexible material, a body side or proximal film 1, an opposed or distal film 2 and an intermediate film 3 having a bottom edge 4 and dividing the pouch into a flatus gas chamber 3a and a faeces chamber 3b.

An elongate drainage portion ending in a drainage opening or outlet 5 defined by the lower ends of films 1 and 2 extends downwards from the main portion of the pouch

A proximal stiffening strip 6 and a distal stiffening strip 7 of a relatively stiff, flexible plastic material such as MYLAR A supplied by Dupont Teijin Films U.S Limited Partnership located in Hopewell, Virginia, USA are attached to the outer surfaces of the lower ends of the films 1 and 2, respectively, and extend immediately adjacent and along the entire length of the drainage opening 5.

The distance of the bottom edge 4 of the intermediate film 3 from the discharge aperture 5 is approximately half the width of strips 6 and 7. A face plate 8 of a skin friendly adhesive barrier material is attached to film 1. The face plate 8 serves to attach the pouch to the peristomal skin surface of a wearer of the ostomy pouch in a manner well known in the art.

The face plate 8 and the film 1 to which the face plate is attached are provided with aligned stoma-receiving apertures 8a or, alternatively, with aligned starter openings which may be enlarged at the time of application by cutting with scissors, all as well known in the art. Also, intermediate film 3 is provided at or near it uppers limits with an opening 8b for allowing flatus gasses to pass from the faeces chamber 3b to the flatus gas chamber 3a while at same time preventing or restraining the passage of faecal material from chamber 3b to chamber 3a, as shown and described in US patent 5,690,623, the disclosure of which is incorporated by reference herein.

A strip 9 of an interlocking means such as Velcro (hook or loop portion), DUOTEC from G. Binder GmbH & Co., Germany (interlocking mushroom elements) or well known repeatedly releasable adhesive coatings, is attached to film 1.

A flap 10 of flexible film material is attached to film 1 by welding or glueing an edge portion 11 of flap 10 to film 2. A strip 12 of a corresponding interlocking means such as Velcro (loop or hook portion), DUOTEC or said repeatedly releasable adhesive coating is attached to the flap 10.

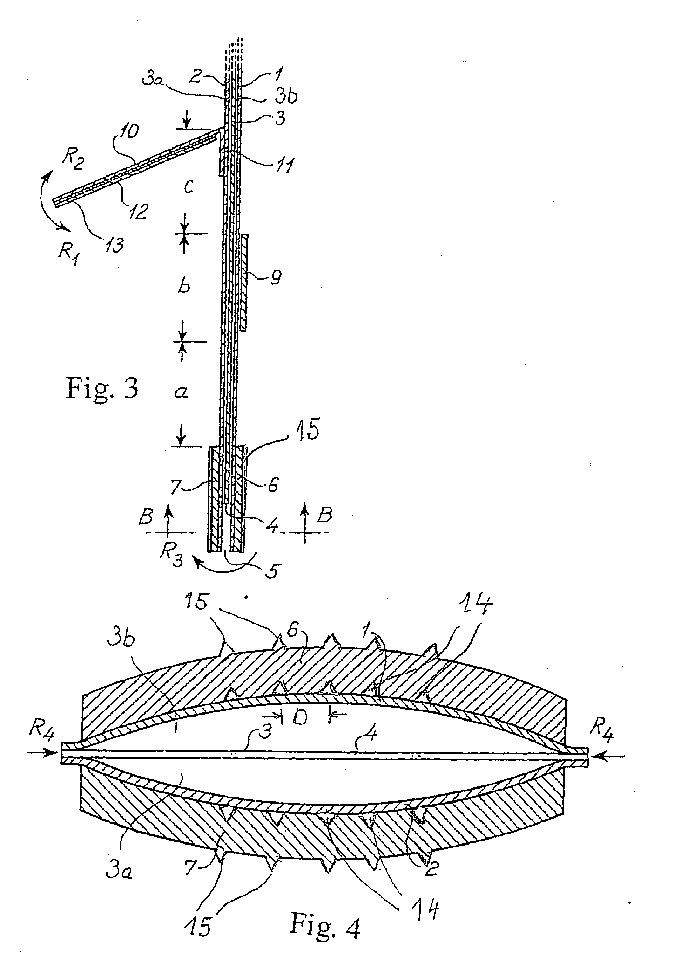

The flap 10 is shown in different positions in Figs. 1, 2 and in Fig. 3, the position of flap 10 shown in Fig. 3 being rotated in the direction R1 compared to the position of flap 10 shown in Figs. 2 and 3 where the flap 10 abuts the film 2, i.e. being rotated as far as possible in the direction R2. The flap 10 has a gripping tab 13 for being gripped by the user's fingers.

Referring now to Figs. 1-4, the strips 6 and 7 are provided with five spaced grooves 14 on the surfaces thereof attached to the films 1 and 2, respectively, and with five corresponding ridges15 provided on the opposed surfaces.

The grooves have a maximum depth of 0.04 mm to 0.06 mm and the ridges have a maximum height of 0.04 mm to 0.06 mm.

The length of the strips is approximately 76 mm, the width is approximately 14 mm, and the thickness is approximately 0.3 mm.

The distance D between adjacent grooves 14 and adjacent ridges 15 is approximately 7.5 mm.

The grooves 14 and ridges 15 are provided by pressing a hard tool with a sharp edge against the strips while the strips are supported by a relatively firm, but resilient surface, for instance rubber having a shore hardness of 80 degrees Shore A.

Alternatively, the grooves and ridges may be provided by scoring the surfaces by moving a sharp point of a tool across the surfaces while applying pressure to the tool and supporting the strips on a relatively firm, but resilient surface.

The grooves may also conceivably be formed by removing material from one surface of the strips, for instance with a toothed rotating blade. In such case no corresponding ridges will be formed.

In use, the discharge portion of the pouch is closed in the following manner after having been emptied through the drainage aperture 5:
The strips 6 and 7 are pressed together to squeeze any remaining matter out from the area between the strips 6 and 7. If any material between the strips 6 and 7 cannot be pressed out, the strips are pulled or flexed apart so as to be able to access said material for removal. The edges of the strips 6 and 7 constituting the discharge aperture 5 are then wiped clean with a tissue.

Thereafter the strips 6 and 7 are rotated clock-wise in the direction of arrow R3 until strip 7 abuts the area a of film 2. Thereafter the strips 6 and 7 are rotated once more clock-wise until strip 6 abuts area b of film 2.

Finally, the strips 6 and 7 are rotated one last time clock-wise such that the coil formed by the strips 6, 7 and 9 as well as the portions of films 1, 2 and 3 involved abuts the area c of film 2 with the strip 9 of Velcro or the like located such that the corresponding strip 12 of Velcro or the like can engage it by rotating the flap 10 in the direction R1 until the two Velcro strips 9 and 12 abut and engage each other for locking said coil in the final closed and secured position of the discharge portion.

In this closed coiled position the upper edge of the coil should preferably fit snugly and tightly in the angle formed between the portion 11 and the rest of the flap 10 such that pressure is exerted thereby on the coil and the portion 11 which is beneficial to the stability of the closure. The rigidity of the strips 6 and 7 is also important for the stability of the closure.

The angle or fold between the portion 11 and the rest of the flap 10 furthermore affords the advantage that the flap 10 in its relaxed state will tend to be in an open position not abutting film 2 in which it is easy to grip when rolling the discharge portion up.

When the pouch is to be drained, the flap 10 is gripped by the tab 13 and pulled such that the strips 9 and 12 are disengaged so that the coil can be unrolled by rotating the strips 6 and 7 three times in the counter clock-wise direction.

The strips 6 and 7 may be used to control the rate of drainage of the pouch by opening and closing the aperture 5 by pressing more or less forcefully on the ends of the strips 6 and 7 in the direction of the arrows R4 so that they flex away from and towards each other, respectively.

Before closing of the pouch by rolling the discharge portion around the strips 6 and 7, the inner surfaces of the walls of the drainage portion must be cleaned to remove any solid matter that could give rise to leaks and bad odours. By pressing the ends of the strips with fingers the strips will flex away from each other and allow easy access to the thereby formed interior channel in the drainage portion.

This function is facilitated by configuring the strips 6 and 7 in the manner shown exaggerated in Fig. 4 of the drawings that shows a longitudinal cross section through the strips 6 and 7 in relaxed state illustrating that the strips have opposed curvatures.

The distance between the bottom edge 4 of intermediate film 3 and the discharge aperture 5 has the advantage that it thereby is easier to separate the three films for cleaning purposes.

Furthermore, by locating the edge 4 between the strips 6 and 7 it is ensured that film 3 is held flat without wrinkles and folded just as many times as the films 1 and 2 such that there is no risk of leaks below the intermediate film 3 between the flatus gas chamber 3a and the faeces chamber 3b in the rolled-up closed position of the drainage portion.

## Claims

1. A drainable ostomy pouch comprising:
a first, proximal (1), and a second, distal, side wall (2) of flexible sheet material sealed to each other for defining a cavity therebetween for receiving human stomal discharge through an aperture in said proximal side wall, and a drainage portion extending downwardly in a longitudinal direction thereof and ending in a drainage opening (5) extending in a transverse direction with respect to said longitudinal direction,
a first, proximal, and a second, distal, elongate, flexible stiffening strip (6, 7) each made of a stiff plastics material and each having a width, a length substantially greater than said width, a first major surface, and a second major surface parallel with and opposed to said first major surface, said stiffening strips (6, 7) extending in said transverse direction in the vicinity of said drainage opening (5), said first major surface of each of said stiffening strips (6, 7) being attached to the outer surface of said first and second side walls (1, 2), respectively, of said drainage portion such that said first major surfaces of said stiffening strips (6, 7) face each other,
**characterized in that**
said first major surface of each of said stiffening strips (6, 7) being provided with at least two spaced grooves (14) extending across said width of said stiffening strips (6, 7).

2. A pouch according to claim 1, wherein each of said stiffening strips (6, 7) is provided with three spaced grooves (14) extending across said width of said stiffening strips (6, 7).

3. A pouch according to claim 1, wherein each of said stiffening strips (6, 7) is provided with four spaced grooves (14) extending across said width of said stiffening strips (6, 7).

4. A pouch according to claim 1, wherein each of said stiffening strips (6, 7) is provided with five spaced grooves (14) extending across said width of said stiffening strips (6, 7).

5. A pouch according to any of the preceding claims, wherein each groove (14) is substantially rectilinear and forms an angle with said transverse direction, said angle being at least 80 degrees.

6. A pouch according to claim 5, wherein said angle is at least 88 degrees, preferably at least 89 degrees.

7. A pouch according to any of the preceding claims, wherein said stiffening strips (6, 7) are generally rectangular.

8. A pouch according to any of the preceding claims, wherein said stiffening strips (6, 7) are generally mutually aligned.

9. A pouch according to any of the preceding claims wherein said grooves (14) extend from one longitudinal edge of said stiffening strip (6, 7) to the opposed longitudinal edge thereof.

10. A pouch according to any of the preceding claims, wherein a mutual distance between one of said grooves (14) and the adjacent groove (14) measured in said transverse direction is between 3 and 12 mm, preferably between 4 and 11 mm, more preferably between 5 and 10 mm, even more preferably between 6 and 9 mm and most preferably between 7 and 8 mm.

11. A pouch according to any of the preceding claims, wherein said stiffening strips (6, 7) are made of MYLAR A PET.

12. A pouch according to any of the claims 1-11, wherein the length of each stiffening strip (6, 7) is between 70 and 80 mm, preferably approximately 76 mm, the width is between 11 and 17 mm, preferably between 12 and 16 mm and most preferably approximately 14 mm, and the thickness is between 0.25 and 0.35 mm, preferably approximately 0.3 mm.

13. A pouch according to any of the preceding claims, wherein said grooves (14) are score lines.

14. A pouch according to claim 13, wherein said score lines are provided by scoring said first major surface with a sharp, hard tool while said second surface is supported by a resilient surface.

15. A pouch according to any of the claims 1-12, wherein said grooves (14) are pressure lines.

16. A pouch according to claim 15, wherein said pressure lines are provided by pressing a hard tool with a sharp edge against said first major surface while said second surface is supported by a resilient surface.

17. A pouch according to any of the preceding claims, wherein said second surface of each of said stiffening strips (6, 7) is provided with a ridge corresponding to a respective one of said grooves and co-extending therewith.

18. A pouch according to any of the precedings claims, wherein each of said stiffening strip (6, 7) is arcuate in said transverse direction such that said first major surface is concave in said transverse direction.

19. A pouch according to any of the preceding claims, wherein the the maximum depth of said grooves relative to said first major surface is from 0.03 mm to 0.07 mm, preferably from 0.04 mm to 0.06mm.

## Patentansprüche

1. Entwässerbarer Ostomiebeutel, enthaltend:
eine erste, proximale (1) und eine zweite, distale Seitenwand (2) aus einem flexiblen Folienmaterial, die miteinander dichtend verbunden sind, um einen Hohlraum, der menschliche Ausscheidungen eines künstlichen Darmausgangs durch eine Öffnung in der proximalen Seitenwand aufnimmt, zwischen sich und einen Drainageabschnitt, der sich in dessen Längsrichtung nach unten erstreckt und in einer Drainageöffnung (5) endet, auszubilden, die in einer Querrichtung im Bezug auf die Längsrichtung verläuft,
einen ersten, proximalen und einen zweiten, distalen, länglichen, flexiblen Aussteifungsstreifen (6, 7), die jeweils aus einem steifen Kunststoffmaterial bestehen und jeweils eine Breite, eine Länge, die im wesentlichen größer als die Breite ist, eine erste Hauptfläche und eine zweite Hauptfläche haben, die zur ersten Hauptfläche parallel ist und dieser gegenüberliegt, wobei die Aussteifungsstreifen (6, 7) in der Querrichtung in der Nähe der Drainageöffnung (5) verlaufen und die erste Hauptfläche jedes Aussteifungsstreifens (6, 7) an der Außenoberfläche der ersten bzw. zweiten Seitenwand (1, 2) des Drainageabschnittes derart angebracht ist, dass die ersten Hauptflächen und die Aussteifungsstreifen (6, 7) einander zugewandt sind,
**dadurch gekennzeichnet, dass**
die erste Hauptfläche jedes Aussteifungsstreifens (6, 7) mit wenigstens zwei beabstandeten Rillen (14) versehen ist, die sich über die Breite der Aussteifungsstreifen (6, 7) erstrecken.

2. Beutel nach Anspruch 1, bei dem jeder der Aussteifungsstreifen (6, 7) mit drei beabstandeten Rillen (14) versehen ist, die sich über die Breite der Aussteifungsstreifen (6, 7) erstrecken.

3. Beutel nach Anspruch 1, bei dem jeder der Aussteifungsstreifen (6, 7) mit vier beabstandeten Rillen (14) versehen ist, die sich über die Breite der Aussteifungsstreifen (6, 7) erstrecken.

4. Beutel nach Anspruch 1, bei dem jeder der Aussteifungsstreifen (6, 7) mit fünft beabstandeten Rillen (14) versehen ist, die sich über die Breite der Aussteifungsstreifen (6, 7) erstrecken.

5. Beutel nach einem der vorhergehenden Ansprüche, bei dem jede Rille (14) im wesentlichen geradlinig ist und einen Winkel mit der Querrichtung einschließt, wobei dieser Winkel wenigstens 80 Grad beträgt.

6. Beutel nach Anspruch 5, bei dem der Winkel wenigstens 88 Grad, vorzugsweise wenigstens 89 Grad beträgt.

7. Beutel nach einem der vorhergehenden Ansprüche, bei dem die Aussteifungsstreifen (6, 7) im wesentlichen rechtwinkelig sind.

8. Beutel nach einem der vorhergehenden Ansprüche, bei dem die Aussteifungsstreifen (6, 7) im wesentlichen wechselseitig ausgerichtet sind.

9. Beutel nach einem der vorhergehenden Ansprüche, bei dem die Rillen (14) von einer Längskante des Verstärkungsstreifens (6, 7) zur gegenüberliegenden Längskante desselben verlaufen.

10. Beutel nach einem der vorhergehenden Ansprüche, bei dem ein wechselseitiger Abstand zwischen einer der Rillen (14) und der benachbarten Rille (14), gemessen in der Querrichtung, zwischen 3 und 12 mm, vorzugsweise zwischen 4 und 11 mm, besser zwischen 5 und 10 mm, stärker bevorzugt zwischen 6 und 9 mm und bestenfalls zwischen 7 und 8 mm beträgt.

11. Beutel nach einem der vorhergehenden Ansprüche, bei dem die Aussteifungsstreifen (6, 7) aus MYLAR A PET bestehen.

12. Beutel nach einem der Ansprüche 1 bis 11, bei dem die Länge jedes Aussteifungsstreifens (6, 7) 70 bis 80 mm, vorzugsweise etwa 76 mm beträgt, die Breite 11 bis 17 mm, vorzugsweise 12 bis 16 mm und bestenfalls etwa 14 mm beträgt und die Dicke 0,25 bis 0,35 mm, vorzugsweise etwa 0,3 mm beträgt.

13. Beutel nach einem der vorhergehenden Ansprüche, bei dem die Rillen (14) Aufreißlinien sind.

14. Beutel nach Anspruch 13, bei dem die Aufreißlinien dadurch hergestellt werden, dass die erste Hauptfläche mit einem scharfen, harten Werkzeug geritzt wird, während die zweite Fläche mit einer elastischen Oberfläche unterstützt wird.

15. Beutel nach einem der Ansprüche 1 bis 12, bei dem die Rillen (14) Drucklinien sind.

16. Beutel nach Anspruch 15, bei dem die Drucklinien dadurch hergestellt werden, dass ein hartes Werkzeug mit einer scharfen Kante gegen die erste Hauptfläche gedrückt wird, während die zweite Oberfläche mit einer elastischen Oberfläche unterstützt wird.

17. Beutel nach einem der vorhergehenden Ansprüche, bei dem die zweite Oberfläche jedes der Aussteifungsstreifen (6, 7) mit einem Grat versehen ist, der mit einer entsprechenden der Rillen korrespondiert und sich mit dieser zusammen erstreckt.

18. Beutel nach einem der vorhergehenden Ansprüche, bei dem jeder der Aussteifungsstreifen (6, 7) in der Querrichtung derart bogenförmig ist, dass die erste Hauptfläche in der Querrichtung konkav ist.

19. Beutel nach einem der vorhergehenden Ansprüche, bei dem die maximale Tiefe der Rillen im Bezug zu der ersten Hauptfläche 0,03 bis 0,07 mm, vorzugsweise 0,04 mm bis 0,06 mm beträgt.

## Revendications

1. Poche de stomie vidable comprenant :
une première paroi latérale proximale (1) et une deuxième paroi latérale distale (2) d'un matériau en feuille flexible, scellées hermétiquement l'une à l'autre pour définir une cavité entre elles, pour recevoir une évacuation de stomie humaine par une ouverture dans ladite paroi latérale proximale, et une partie de drainage s'étendant vers le bas dans une direction longitudinale de celle-ci et se terminant dans une ouverture de drainage (5) s'étendant dans une direction transversale par rapport à ladite direction longitudinale,
une première bande de raidissement allongée flexible proximale et une deuxième bande de raidissement allongée flexible distale (6, 7) constituées respectivement d'une matière plastique rigide et chacune ayant une largeur, une longueur substantiellement supérieure à ladite largeur, une première surface principale et une deuxième surface principale parallèle et opposée à ladite première surface principale, lesdites bandes de raidissement (6, 7) s'étendant dans ladite direction transversale à proximité de ladite ouverture de drainage (5), ladite première surface principale de chacune desdites bandes de raidissement (6, 7) étant fixée à la surface extérieure desdites première et deuxième parois latérales (1, 2), respectivement, de ladite partie de drainage de telle sorte que lesdites premières surfaces principales desdites bandes de raidissement (6, 7) soient face à face,
**caractérisée en ce que**
ladite première surface principale de chacune desdites bandes de raidissement (6, 7) est dotée d'au moins deux sillons espacés (14) s'étendant sur ladite largeur desdites bandes de raidissement (6, 7).

2. Poche selon la revendication 1, dans laquelle chacune desdites bandes de raidissement (6, 7) est dotée de trois sillons espacés (14) s'étendant sur ladite largeur desdites bandes de raidissement (6, 7).

3. Poche selon la revendication 1, dans laquelle chacune desdites bandes de raidissement (6, 7) est dotée de quatre sillons espacés (14) s'étendant sur ladite largeur desdites bandes de raidissement (6, 7).

4. Poche selon la revendication 1, dans laquelle chacune desdites bandes de raidissement (6, 7) est dotée de cinq sillons espacés (14) s'étendant sur ladite largeur desdites bandes de raidissement (6, 7).

5. Poche selon l'une quelconque des revendications précédentes, dans laquelle chaque sillon (14) est substantiellement rectiligne et forme un angle avec ladite direction transversale, ledit angle étant d'au moins 80 degrés.

6. Poche selon la revendication 5, dans laquelle ledit angle est d'au moins 88 degrés, de préférence, d'au moins 89 degrés.

7. Poche selon l'une quelconque des revendications précédentes, dans laquelle lesdites bandes de raidissement (6, 7) sont généralement rectangulaires.

8. Poche selon l'une quelconque des revendications précédentes, dans laquelle lesdites bandes de raidissement (6, 7) sont généralement alignées respectivement.

9. Poche selon l'une quelconque des revendications précédentes, dans laquelle lesdits sillons (14) s'étendent d'un bord longitudinal desdites bandes de raidissement (6, 7) au bord longitudinal opposé de celles-ci.

10. Poche selon l'une quelconque des revendications précédentes, dans laquelle une distance respective entre l'un desdits sillons (14) et le sillon adjacent (14) mesurée dans ladite direction transversale se situe entre 3 et 12 mm, de préférence, entre 4 et 11 mm, de manière davantage préférée, entre 5 et 10 mm, de manière encore davantage préférée entre 6 et 9 mm et de manière préférée entre toutes, entre 7 et 8 mm.

11. Poche selon l'une quelconque des revendications précédentes, dans laquelle lesdites bandes de raidissement (6, 7) sont constituées de PET MYLAR A.

12. Poche selon l'une quelconque des revendications 1 à 11, dans laquelle la longueur de chaque bande de raidissement (6, 7) se situe entre 70 et 80 mm, de préférence, est d'environ 76 mm, la largeur se situe entre 11 et 17 mm, de préférence entre 12 et 16 mm et de manière préférée entre toutes, d'environ 14 mm et l'épaisseur se situe entre 0,25 et 0,35 mm, de préférence, d'environ 0,3 mm.

13. Poche selon l'une quelconque des revendications précédentes, dans laquelle lesdits sillons (14) sont des lignes de repère.

14. Poche selon la revendication 13, dans laquelle lesdites lignes de repère sont constituées en rainurant ladite première surface principale à l'aide d'un outil acéré dur alors que ladite deuxième surface est soutenue par une surface élastique.

15. Poche selon l'une quelconque des revendications 1 à 12, dans laquelle lesdits sillons (14) sont des conduites de pression.

16. Poche selon la revendication 15, dans laquelle lesdites conduites de pression sont constituées en appuyant un outil dur présentant une arête acérée contre ladite première surface majeure alors que ladite deuxième surface est soutenue par une surface élastique.

17. Poche selon l'une quelconque des revendications précédentes, dans laquelle ladite deuxième surface de chacune desdites bandes de raidissement (6, 7) est dotée d'une strie correspondant respectivement à l'un desdits sillons et s'étendant conjointement à celui-ci.

18. Poche selon l'une quelconque des revendications précédentes, dans laquelle chacune desdites bandes de raidissement (6, 7) est arquée dans ladite direction transversale de telle sorte que ladite première surface principale soit concave dans ladite direction transversale.

19. Poche selon l'une quelconque des revendications précédentes, dans laquelle ladite profondeur maximale desdits sillons par rapport à ladite première surface principale est de 0,03 mm à 0,07 mm, de préférence, de 0,04 mm à 0,06 mm.
